# FASCICULE DE BREVET EUROPEEN

(11) **EP 2 619 281 B1**
(45) Date de publication et mention de la délivrance du brevet: **09.11.2016**
(21) Numéro de dépôt: 11764800.6
(22) Date de dépôt: 16.09.2011
(51) Int. Cl.: C09K 11/06, C07H 17/075, C12Q 1/34, G01N 21/64

(54) **COUMARINES SULFONATÉES, LEUR SYNTHÈSE, SUBSTRATS FLUOROGÉNIQUES RÉSULTANT D'UN GREFFAGE DE CES COUMARINES SUR DES SUCRES, LE PROCÉDÉ D'OBTENTION DE CES SUBSTRATS, AINSI QUE LEURS APPLICATIONS**
SULFONIERTE CUMARINE, IHRE SYNTHESE, FLUOROGENE SUBSTRATE AUS DER PFROPFUNG DIESER CUMARINE AUF ZUCKER, VERFAHREN ZUR HERSTELLUNG DIESER SUBSTRATE UND IHRE VERWENDUNG
SULFONATED COUMARINS, SYNTHESIS THEREOF, FLUOROGENIC SUBSTRATES RESULTING FROM GRAFTING SAID COUMARINS ONTO SUGARS, METHOD FOR PREPARING SAID SUBSTRATES, AND USES THEREOF

(30) Priorité: 21.09.2010 FR 1003759
(43) Date de publication de la demande: 31.07.2013
(60) Demande divisionnaire: 16181401.7
(73) Titulaire: ETS J. Soufflet, 10402 Nogent-sur-Seine (FR); Université de Strasbourg, 67000 Strasbourg (FR)
(72) Inventeur: DREVELLE, Antoine, F-10100 Romilly (FR); LADAME, Sylvain, Hoddesdon EN119DQ (GB); NAJAH, Majdi, F-10400 Fontaine Mâcon (FR); MAYOT, Estelle, F-67300 Schiltigheim (FR)
(74) Mandataire: Regimbeau
(86) Numéro de dépôt international: PCT/FR2011/000504
(87) Numéro de publication internationale: WO 2012/038614

(56) Documents cités:
- GB-A- 2 319 250

## Description

La présente invention concerne des substrats fluorogéniques résultant d'un greffage de coumarines sulfonatées sur des sucres, le procédé d'obtention de ces substrats, ainsi que leurs applications.

Différentes méthodes de fabrication de telles coumarines sulfonatées sont connues, par exemple par le brevet anglais GB9723365 au nom de MOLECULAR PROBES qui décrit la synthèse de dérivés de la 6,8-difluoro-7-hydroxycoumarine.

C'est ainsi que ce brevet cite notamment la 6,8-difluoro-7-hydroxycoumarin-4-méthanesulfonate de sodium (composé n° 37).

Le brevet GB9723365 décrit aussi des composés résultant d'un remplacement du groupe hydroxyle par un reste de sucre, ce dernier étant donc greffé sur la coumarine sulfonatée. La reproduction du procédé de greffage décrit dans le brevet précité n'est pas valable pour les coumarines sulfonatées car elles ne sont pas solubles dans les conditions décrites.

Un objectif de la présente invention est de fournir des substrats fluorogéniques résultant d'un greffage de coumarines sulfonatées substituées sur des sucres grâce à un procédé de greffage reproductible avec différents sucres et différentes coumarines sulfonatées substituées. On entend dans la présente description par substrat fluorogénique un substrat non fluorescent dont l'hydrolyse de la liaison entre le sucre et la coumarine libère la coumarine, qui est en elle-même fluorescente.

Un autre objectif de la présente invention concerne une méthode de détection d'activités glycosidases (EC3.2.1) sur des extraits enzymatiques purifiés ou non ou sur des microorganismes ou sur des cellules.

Une famille de coumarines sulfonatées substituées de formule générale (I) 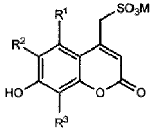 dans laquelle :
- R¹ représente H, ou OH, ou un radical alkyle de C₁ à C₆ substitué ou non, linéaire ou ramifié, ou -COR⁴, ou -COOR⁴, ou -CONHR⁴
- R² représente H, ou un halogène, notamment le fluor, ou un radical alkylde de C₁ à C₆ substitué ou non, linéaire ou ramifié, ou -COR⁴, ou -COOR⁴, ou -CONHR⁴
- R¹ et R² pouvant former ensemble un cycle, tel qu'un aryle ou un furane, substitué ou non
- R³ représente H, ou un halogène, notamment le fluor, ou un radical alkyle de C₁ à C₆ substitué ou non, linéaire ou ramifié, ou -COR⁴, ou -COOR⁴, ou -CONHR⁴
- avec R⁴ étant H, ou un radical alkyle de C₁ à C₆ substitué ou non, linéaire ou ramifié ou un aryle, substitué ou non
- M représente Na ou K,
est décrite.

La présente invention concerne de nouveaux substrats ayant pour formule générale (II) 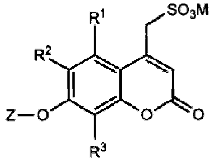 dans laquelle :
- R¹ représente H, ou OH, ou un radical alkyle de C₁ à C₆ substitué ou non, linéaire ou ramifié, ou -COR⁴, ou -COOR⁴, ou -CONHR⁴
- R² représente H, ou un halogène, notamment le fluor, ou un radical alkyl de C₁ à C₆ substitué ou non, linéaire ou ramifié, ou -COR⁴, ou -COOR⁴, ou -CONHR⁴
- R¹ et R² pouvant former ensemble un cycle, tel qu'un aryle ou un furane, substitué ou non
- R³ représente H, ou un halogène, notamment le fluor, ou un radical alkyle de C₁ à C₆ substitué ou non, linéaire ou ramifié, ou -COR⁴, ou -COOR⁴, ou -CONHR⁴
- avec R⁴ étant H, ou un radical alkyle de C₁ à C₆ substitué ou non, linéaire ou ramifié ou un aryle, substitué ou non
- M représente Na ou K
- Z représente un sucre choisi parmi les sucres suivants : cellobiose, xylobiose, maltose, saccharose, glucose, xylose, galactose, arabinose, xylane, glucane, xylotriose, maltotriose, cellotriose, xylotétraose ou un mélange de certains d'entre eux.

L'invention sera mieux comprise à la lecture de la description qui va suivre, référence étant faite aux composés numérotés 1 à 24 suivants : 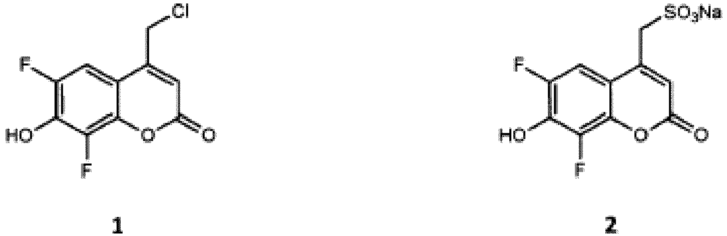 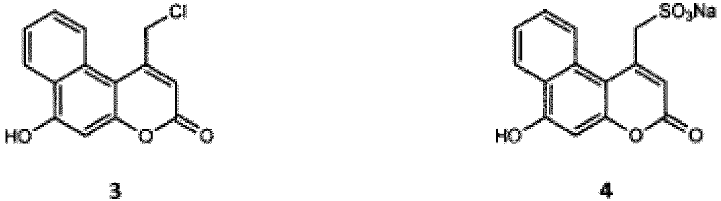 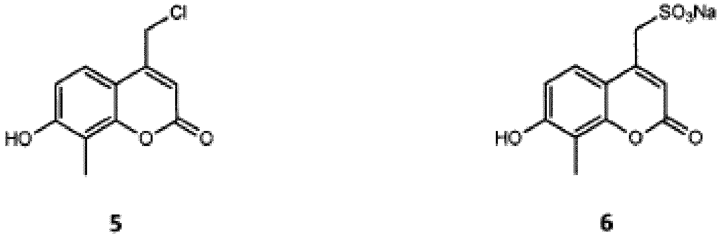 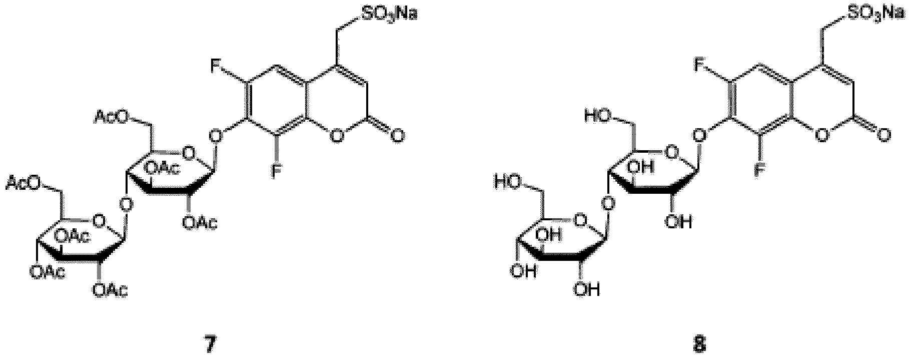 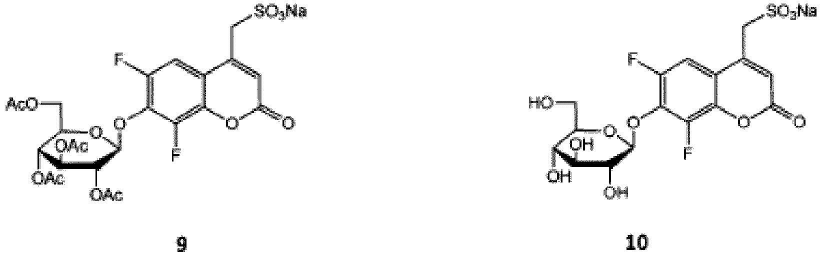 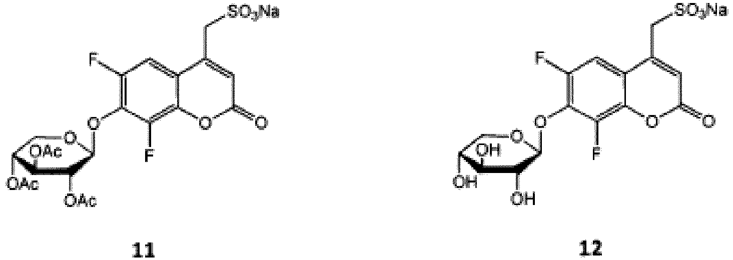 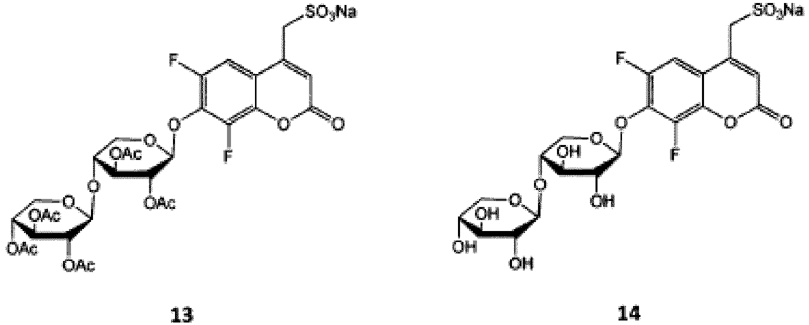 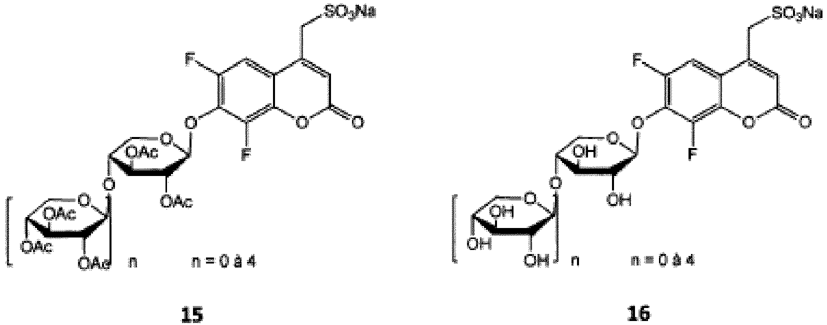 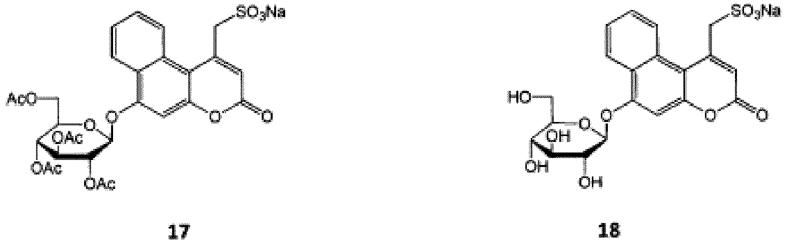 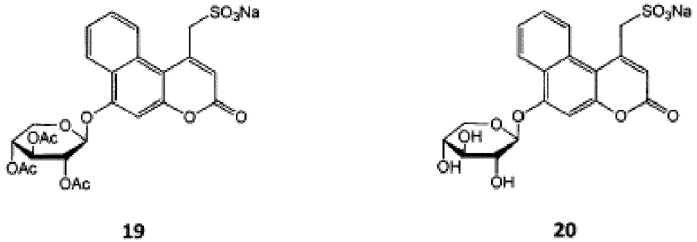 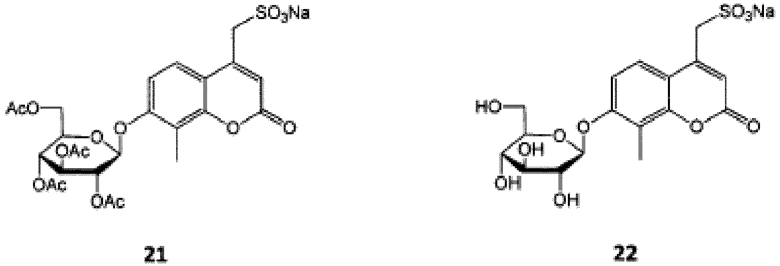 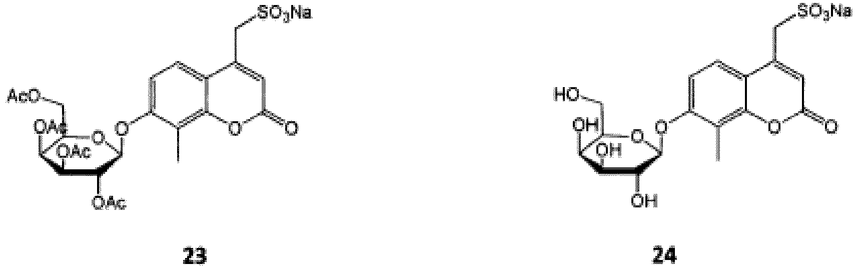 parmi lesquels les composés **2, 4** et **6** sont des coumarines sulfonatées substituées entrant dans la formule générale (I) et où les composés **8, 10, 12, 14, 16, 18, 20, 22 et 24** sont des exemples de substrats entrant dans la formule générale (II).

Différents exemples de synthèse de ces coumarines et substrats vont maintenant être donnés, à titre indicatif et nullement limitatif.

### Exemple 1. Synthèse de 4-chlorométhyl-6,8-difluoro-7-hydroxycoumarine (1)

Une solution de 2,4-difluororésorcinol (0,8 g) dans 8 ml d'acide méhylsulfonique est introduit dans un ballon puis 1,3 équivalent (963 µl) de 4-chloroacétoacétate d'éthyle sont ajoutés goutte à goutte. Après 3h d'agitation à température ambiante, le mélange est refroidi à 0°C puis 100 ml d'eau sont additionnés. Le résidu est alors filtré, séché puis lavé à l'éther diéthylique pour donner 0,34 g (25%) du composé **1.**
RMN¹H (MeOD) δ (ppm) 7,42 (d, 1H) ; 6,05 (s, 1H) ; 4,81 (s, 2H)

### Exemple 2. Synthèse de 6,8-difluoro-7-hydroxycoumarin-4-méthanesulfonate de sodium (2)

Une solution de 320 mg du composé 1 dissous dans 25 ml d'éthanol est introduite dans un ballon, puis une solution de sulfite de sodium (1,1 équivalent, 185 mg) dans 6 ml d'eau est additionnée. Le mélange est porté à reflux et l'évolution de la réaction est suivie par CCM (Chromatographie sur Couche Mince). Après 40h de chauffage, le mélange est refroidi à 0°C. La solution est filtrée puis le filtrat est évaporé sous pression réduite pour donner un solide jaune.

Le solide obtenu est purifié une première fois par chromatographie sur colonne de silice inverse pré-packée (éluant : eau pure, cartouche *Puriflash Interchrom* C18) puis ensuite avec un appareil de purification *Biotage*-*IsoleraOne* (éluant : eau pure, cartouche SNAP C18 120 g, débit d'élution : 25 ml/min).

Après évaporation de l'eau sous pression réduite, le produit 2 est obtenu pur avec un rendement de 40%.
RMN ¹H (D₂O) δ (ppm) 7,28 (d, 1H) ; 6,18 (s, 1H) ; 4,32 (s 2H).
RMN ¹³C (D₂O) δ (ppm) 163,7 ; 153,1 (dd) ; 150,5 (t) ; 149,0 ; 142,2 (dd) ; 140,7 (m) ; 109,2 ; 105,3 (d) ; 103,6 (d) ; 52,7.
Maximum excitation/émission = 370/470 nm (10 µM dans du tampon phosphate 50 mM, NaCl 150 mM, pH = 7,5).

### Exemple 3. Synthèse de 5,6-benzo-4-chlorométhyl-7-hydroxycoumarine (3)

Une solution de 1,3-dihydroxynaphtalène (1 g) dans 10 ml d'acide méthanesulfonique est introduit dans un ballon puis 1,3 équivalent (1,10 ml) de 4-chloroacétoacétate d'éthyle sont ajoutés goutte à goutte. Après 4 jours d'agitation à température ambiante, le mélange est refroidi à 0°C puis 50 ml d'eau sont additionnés. Le résidu est alors filtré, lavé à l'eau et séché pour donner 1,30 g (80%) du composé 3.
RMN ¹H (DMSO) δ (ppm) 8,47 (d, 1H) ; 8,32 (d, 1H) ; 7,75 (t, 1H) ; 7,60 (t, 1H) ; 6,88 (s, 1H) ; 6,58 (s, 1H) ; 5,33 (s, 2H).

### Exemple 4. Synthèse de 5,6-benzo-7-hydroxycoumarin-4-méthanesulfonate de sodium (4)

Une solution de 0,7 g du composé 3 dissous dans 50 ml d'éthanol est introduite dans un ballon, puis une solution de sulfite de sodium (1,1 équivalent, 370 mg) dans 13 ml d'eau est additionnée. Le mélange est porté à reflux et l'évolution de la réaction est suivie par CCM. Après 20h de chauffage, le mélange est refroidi à 0°C. La solution est filtrée sur un entonnoir Büchner puis le filtrat est évaporé sous pression réduite pour donner le composé **4** brut sous forme d'un solide jaune avec un rendement de 77%.

Le solide obtenu est purifié une première fois par chromatographie sur colonne de silice inverse pré-packée (éluant : eau pure, cartouche *Puriflash Interchrom* C18) puis ensuite avec un appareil de purification *Biotage*-*IsoleraOne* (éluant : 10% acétonitrile / 90% eau, cartouche SNAP C18 120 g, débit d'élution : 25 ml/min).

Après évaporation des solvants sous pression réduite, le produit **4** est obtenu pur avec un rendement de 31%.
RMN ¹'H (D20) δ (ppm) 8,26 (d, 1H) ; 8,02 (d, 1H) ; 7,56 (t, 1H) ; 7,44 (t, 1H) ; 6,32 (s, 1H) ; 6,11 (s, 1H) ; 4,45 (s, 2H).
Maximum excitation/émission = 420/510 nm (100 µM dans du tampon phosphate 50 mM, NaCl 150 mM, pH = 7,5).

### Exemple 5. Synthèse de 4-chlorométhyl-7-hydroxy-8-méthylcoumarine (5)

Une solution de 2-méthylrésorcinol (1 g) dans 10 ml d'acide méthylsulfonique est introduit dans un ballon puis 1,3 équivalent (1,42 ml) de 4-chloroacétoacétate d'éthyle sont ajoutés goutte à goutte. Après 1 nuit d'agitation à température ambiante, le mélange est refroidi à 0°C puis 50 ml d'eau sont additionnés. Le résidu est alors filtré, séché puis lavé à l'éther diéthylique pour donner 1,63 g (90%) du composé **5.**
RMN ¹H (DMSO) δ (pm) 7,52 (d, 1H) ; 6,90 (d, 1H) ; 6,41 (s, 1H) ; 4,93 (s, 2H) ; 2,16 (s, 3H). RMN ¹³C (DMSO) δ (ppm) 160,8 ; 159,7 ; 153,6 ; 151,7 ; 123,5 ; 112,3 ; 111,6 ; 111,1 ; 109,8 ; 41,9 ; 8,4.

### Exemple 6. Synthèse de 4-chlorométhyl-7-hydroxy-8-méthylcoumarin-4-méthanesulfonate de sodium (6)

Une solution de 1 g du composé **5** dissous dans 90 ml d'éthanol est introduite dans un ballon, puis une solution de sulfite de sodium (1,1 équivalent, 617 mg) dans 22 ml d'eau est additionnée. Le mélange est porté à reflux. Après 20h de chauffage, le mélange est refroidi à 0°C. La solution est filtrée sur un entonnoir Büchner, le résidu beige obtenu est lavé à l'éthanol, quant au filtrat, il est évaporé sous pression réduite pour donner un solide jaune. Ce solide est lavé à l'éthanol et à l'éther diéthylique. Les deux solides obtenus sont rassemblés pour donner 1 g (80%) de composé **6** brut.

Le composé **6** brut est ensuite purifié par plusieurs chromatographies sur colonne de silice inverse pré-packée (éluant : eau pure, cartouche *Puriflash Interchrom* C18) successives pour donner le produit **6** pur avec un rendement de 23%.
RMN ¹H (D₂O) δ (ppm) 7,32 (d, 1H) ; 6,69 (d, 1H) ; 6,15 (s, 1H) ; 4,19 (s, 2H) ; 1,97 (s, 3H). RMN ¹³C (D₂O) δ (ppm) 164,2 ; 158,5 ; 148,9 ; 123,9 ; 112,6 ; 112,2 ; 111,8 ; 111,3 ; 52,4 ; 7,3.
Maximum excitation/émission = 340/485 nm (10 µM dans du tampon phosphate 50 mM, NaCl 150 mM, pH = 7,5).

### Exemple 7. Synthèse du substrat β-D-cellobioside 6,8-difluoro-7- hydroxycoumarin-4-méthanesulfonate de sodium (8)

### Bromation du sucre protégé :

Sous atmosphère d'azote, 1 g d'acéto-α-D-cellobiose et 12,5 ml de DCM (dichlorométhane) anhydre sont introduits dans un ballon. Le mélange est refroidi à 0°C puis 15 ml d'une solution d'acide bromhydrique en solution à 33% dans l'acide acétique sont ajoutés goutte à goutte. Après 5h de réaction à 0°C, 25 ml de DCM sont additionnés, puis le mélange est encore agité pendant 10min. Une solution de carbonate de potassium est ensuite ajoutée précautionneusement jusqu'à disparition du dégagement de C02, le milieu est laissé sous agitation pendant 20min. Après décantation, la phase organique est récupérée, la phase aqueuse est extraite 3 fois au DCM, puis les phases organiques sont combinées, séchées sur MgSO₄ et évaporées sous pression réduite. L'acétobromo-α-D-cellobiose est obtenu sous forme d'un solide blanc avec un rendement de 62%. Le produit ainsi obtenu est instable, et il convient soit de l'utiliser directement, soit de le conserver à -20°C.
RMN ¹'H (CDCl₃) δ (ppm) 6,67 (m, 1H) ; 5,55 (t, 1H) ; 5,13 (m, 2H) ; 4,95 (t, 1H) ; 4,78 (m, 1H) ; 4,55 (m, 2H) ; 4,38 (m, 1H) ; 4,20 (m, 2H) ; 4,07 (m, 1H) ; 3,84 (m, 1H) ; 3,68 (m, 1H) ; 2,09 (m, 21H).

### Greffage du sucre sur la coumarine :

Sous atmosphère d'azote et protection de la lumière, 40 mg de coumarine 2 dilués dans 5 ml de DMF (diméthylformamide) anhydre et 150 mg de carbonate d'argent sont introduits dans un ballon. 4 équivalents d'acétobromo-α-D-cellobiose (350 mg) sont dissous dans 10 ml de DMF anhydre. Cette solution est ensuite ajoutée très lentement au mélange précédent. Après une nuit d'agitation à température ambiante, la solution est filtrée sur célite et le filtrat est évaporé sous pression réduite. Le solide obtenu est enfin purifié par chromatographie sur colonne de silice (éluant : 20% MeOH / 80% DCM). Le composé 7 est obtenu avec un rendement de 4,8%.

### Déprotection des fonctions de type acétal :

Sous atmosphère d'azote, 40 mg du composé 7 et 7,5 ml de méthanol anhydre sont introduits dans un ballon. Le mélange est refroidi à 0°C puis une solution de 16 mg de méthylate de sodium dans 5 ml de méthanol est ajoutée goutte à goutte. L'évolution de la réaction est suivie par LC/MS. Après 1h d'agitation, le milieu est neutralisé par addition d'une solution diluée d'acide citrique. Après une agitation supplémentaire de 20 min, le solvant est évaporé sous pression réduite pour donner une huile jaune translucide.

Le résidu obtenu est purifié une première fois par chromatographie sur colonne de silice inverse pré-packée (éluant : eau pure, cartouche *Puriflash Interchrom* C18) puis ensuite avec une HPLC (*High-Pressure Liquid Chromatography*).

### Méthode HPLC :

- 0 min→ 10 min ; 1,5 ml/min : 100% eau
- 10 min→ 19 min ; 1,5 ml/min : 90% eau / 10% acétonitrile
- 19 min→ 23 min ; 1,5 ml/min : 100% acétonitrile
- 23 min→ 25 min ; 1,5 ml/min : 100% eau

Caractéristiques de la colonne : Colonne Hypersil ; Gold Phenyl ; Thermofisher 5 ; longueur 250 mm ; diamètre 4,6 mm
Les injections sont de 80 µl et le produit sort uniquement dans la phase eau dans l'intervalle : 0min → 10min, suivi de très près par la coumarine libre présente en solution.

Après lyophilisation, le produit **8** est obtenu pur sous forme d'un solide blanc avec un rendement de 30%.
RMN ¹H (D₂O) δ (ppm) 7,55 (d, 1H) ; 6,60 (s, 1H) ; 5,17 (d, 1H) ; 4,45 (d, 1H) ; 4,38 (s, 2H) ; 3,9-3,2 (m, 12H).

### Exemple 8. Synthèse du substrat β-D-glucoside 6,8-difluoro-7-hydroxycoumarin-4-méthanesulfonate de sodium (10)

### Greffage du sucre sur la coumarine :

Sous atmosphère d'azote et protection de la lumière, 100 mg de coumarine 2 dilués dans 5 ml de DMF anhydre et 5 équivalents de carbonate d'argent sont introduits dans un ballon. 5 équivalents d'acétobromo-α-D-glucose dont dissous dans 5 ml de DMF anhydre. Cette solution est ensuite ajoutée très lentement au mélange précédent. L'évolution de la réaction est suivie par LC/MS. Après une nuit d'agitation à température ambiante, la solution est filtrée sur célite et le filtrat est évaporé sous pression réduite.
Le solide obtenu est enfin purifié par chromatographie sur colonne de silice (éluant : 20% MeOH / 80% DCM). Le composé **9** est obtenu avec un rendement de 37%.
RMN ¹H (MeOD) δ (ppm) 7,73 (d, 1H) ; 6,63 (s, 1H) ; 5,4-5,1 (m, 4H) ; 4,4-4,1 (m, 2H) ; 4,34 (s, 2H) ; 4,04 (m, 1H) ; 2,1 (m, 12H).

### Déprotection des fonctions de type acétal :

Sous atmosphère d'azote, 45 mg du composé **9** et 10 ml de méthanol anhydre sont introduits dans un ballon. Le mélange est refroidi à 0°C puis 350 µl d'une solution de méthylate de sodium à 1% (g/g) dans le méthanol est ajoutée goutte à goutte. L'évolution de la réaction est suivie par LC/MS. Après 2h30 d'agitation, de l'Amberlite IR120 est ajouté jusqu'à neutralisation du milieu. Après filtration de l'Amberlite, le filtrat est évaporé sous pression réduite.

Le résidu obtenu est purifié une première fois par chromatographie sur colonne de silice inverse pré-packée (éluant : eau pure, cartouche *Puriflash Interchrom* C18) puis ensuite avec un appareil de purification *Biotage*-*IsoleraOne* (éluant : eau pure, cartouche SNAP C18 12 g, débit d'élution : 5 ml/min).

Après lyophilisation, le produit **10** est obtenu pur avec un rendement de 54%.
RMN ¹H (D₂O) δ (ppm) 7,52 (d, 1H) ; 6,54 (s, 1H) ; 5,11 (d,1H) ; 4,32 (s, 2H) ; 3,78 (m, 1H) ; 3,63 (m, 1H) ; 3,6-3,4 (m, 4H).

### Exemple 9. Synthèse du substrat β-D-xyloside 6,8-difluoro-7-hydroxycoumarin-4-méthanesulfonate de sodium (12)

### Bromation du sucre protégé :

Sous atmosphère d'azote, 500 mg d'acéto-β-D-xylopyranose et 10 ml de DCM sont introduits dans un ballon. Le mélange est refroidi à 0°C puis 5 équivalents d'acide bromhydrique en solution à 33% dans l'acide acétique dont ajoutés au goutte à goutte. Après 24h de réaction, 15 ml de DCM sont additionnés, le mélange est refroidi à 0°C puis 20 ml d'eau glacée sont ajoutés. Après séparation des deux phases, la phase organique est lavée 3 fois avec une solution saturée en NaHCO₃ puis 3 fois avec de l'eau. Après séchage sur MgSO₄ et filtration, le filtrat est évaporé sous pression réduite. L'acétobromo-α-D-xylose obtenu est ensuite directement introduit dans l'étape suivante.

### Greffage du sucre sur la coumarine :

Sous atmosphère d'azote et protection de la lumière, 100 mg de coumarine 2 dilués dans 8 ml de DMF anhydre et 5 équivalents de carbonate d'argent sont introduits dans un ballon. 5 équivalents d'acétobromo-α-D-xylose sont dissous dans 6 ml de DMF anhydre. Cette solution est ensuite ajoutée très lentement au mélange précédent. L'évolution de la réaction est suivie par LC/MS. Après une nuit d'agitation à température ambiante, la solution est filtrée sur célite et le filtrat est évaporé sous pression réduite.
Le solide obtenu est enfin purifié par chromatographie sur colonne de silice (éluant : 20% MeOH / 80% DCM). Le composé **11** est obtenu pur avec un rendement de 34%.
RMN ¹'H (MeOD/D₂O) δ (ppm) 7,70 (d, 1H) ; 6,66 (s, 1H) ; 5,46 (d, 1H) ; 5,25 (m, 2H) ; 5,04 (m, 1H) ; 4,37 (s, 2H) ; 4,32 (m, 1H) ; 3,70 (m, 1 H) ; 2,13 (m, 9H).

### Déprotection des fonctions de type acétal :

Sous atmosphère d'azote, 62 mg du composé **11** et 15 ml de méthanol anhydre sont introduits dans un ballon. Le mélange est refroidi à 0°C puis 500 µl d'une solution de méthylate de sodium à 1% (g/g) dans le méthanol est ajoutée goutte à goutte. L'évolution de la réaction est suivie par LC/MS. Après 1h30 d'agitation, de l'Amberlite IR120 est ajouté jusqu'à neutralisation du milieu. Après filtration de l'Amberlite, le filtrat est évaporé sous pression réduite.

Le résidu obtenu est purifié une première fois par chromatographie sur colonne de silice inverse pré-packée (éluant : eau pure, cartouche *Puriflash Interchrom* C18) puis ensuite avec un appareil de purification *Biotage*-*IsoleraOne* (éluant : eau pure, cartouche SNAP C18 30 g, débit d'élution : 15 ml/min).

Après lyophilisation, le produit **12** est obtenu pur avec un rendement de 62%.
RMN ¹H (D₂O) δ (ppm) 7,64 (d,1H) ; 6,66 (s, 1H) ; 5,14 (d, 1H) ; 4,43 (s, 2H) ; 4,02 (m, 1H) ; 3,5-3,7 (m, 3H) ; 3,35 (m, 1H).

### Exemple 10. Synthèse du substrat β-D-xylobioside 6,8-difluoro-7-hydroxycoumarin-4-méthanesulfonate de sodium (14)

### Bromation du sucre protégé :

Les conditions opératoires sont identiques à celles utilisées dans l'exemple 9 en utilisant l'acéto-D-xylobiose comme produit de départ. L'acétobromo-α-D-xylobiose obtenu est ensuite directement introduit dans l'étape suivante.

### Greffage du sucre sur la coumarine :

Sous atmosphère d'azote et protection de la lumière, 60 mg de coumarine **2** dilués dans 5 ml de DMF anhydre et 5 équivalents de carbonate d'argent sont introduits dans un ballon. 3 équivalents d'acétobromo-α-D-xylobiose dont dissous dans 5 ml de DMF anhydre. Cette solution est ensuite ajoutée très lentement au mélange précédent. Après une nuit d'agitation à température ambiante, la solution est filtrée sur célite et le filtrat est évaporé sous pression réduite.

Le solide obtenu est purifié deux fois successivement par chromatographie sur colonne de silice (éluant : gradient de 10% MeOH à 20% MeOH dans du DCM). Le composé **13** est obtenu pur avec un rendement de 18%.
RMN ¹H (MeOD) δ (ppm) 7,70 (d, 1H) ; 6,61 (s, 1H) ; 5,38 (d, 1H) ; 5,16 (m, 3H) ; 4,92 (m, 1H) ; 4,78 (m, 2H) ; 4,32 (s, 2H) ; 4,18 (m, 1H) ; 4,10 (m, 1H) ; 4,03 (m, 1H) ; 3,54 (m, 2H) ; 2,09 (m, 15H).

### Déprotection des fonctions de type acétal :

Sous atmosphère d'azote, 27 mg du composé **13** et 7 ml de méthanol anhydre sont introduits dans un ballon. Le mélange est refroidi à 0°C puis 200 µl d'une solution de méthylate de sodium à 1% (g/g) dans le méthanol est ajoutée goutte à goutte. L'évolution de la réaction est suivie par LC/MS. Après 3h30 d'agitation, de l'Amberlite IR120 est ajouté jusqu'à neutralisation du milieu. Après filtration de l'Amberlite, le filtrat est évaporé sous pression réduite. Le résidu est repris dans de l'eau puis la solution est filtrée à 0,45 µm, le filtrat est de nouveau évaporé sous pression réduite.

Le résidu obtenu est purifié avec un appareil de purification *Biotage*-*IsoleraOne* (éluant : 5% acétonitrile dans l'eau pure, cartouche SNAP C18 12 g, débit d'élution : 3 ml/min).

Après évaporation des solvants, le produit **14** est obtenu pur avec un rendement de 40%.
RMN ¹H (D₂O) δ (ppm) 7,56 (d, 1H) ; 6,58 (s, 1H) ; 5,11 (d, 1H) ; 4,40 (d, 1H) ; 4,36 (s, 2H) ; 4,10 (m, 1H) ; 3,91 (m, 1H) ; 3,82 (m, 1H) ; 3,7-3,5 (m, 3H) ; 3,40 (m, 2H) ; 3,21 (m, 2H).

### Exemple 11. Synthèse du substrat β-D-xylopolyoside 6,8-difluoro-7- hydroxycoumarin-4-méthanesulfonate de sodium (16)

### Acétylation du xylopolyose, à savoir un mélange de xylobiose, xylotriose, ... :

Sous atmosphère d'azote, 300 mg de xylopolyose, quelques grains de 4-diméthylaminopyridine, 28 ml de DCM anhydre et 9 ml de pyridine anhydre sont introduits dans un ballon. Le mélange est refroidi à 0°C puis 3 ml d'anhydride d'acétique sont additionnés goutte à goutte. Le milieu est ensuite laissé sous agitation pendant une nuit. Le mélange est ensuite lavé 3 fois à l'eau, séché sur MgSO₄ puis évaporé sous pression réduite.

L'analyse par LC/MS montre que le mélange est constitué d'acéto-xylopolyose avec n = 1 à 5.

### Bromation du sucre protégé :

Les conditions opératoires sont identiques à celles utilisées dans l'exemple 9 en utilisant 102 mg d'acéto-xylopolyose comme produit de départ. L'acétobromo-α-D-xylopolyose obtenu est ensuite directement introduit dans l'étape suivante.

### Greffage du sucre sur la coumarine :

Sous atmosphère d'azote et protection de la lumière, 60 mg de coumarine 2 dilués dans 5 ml de DMF anhydre et 5 équivalents de carbonate d'argent sont introduits dans un ballon. 106 mg d'acétobromo-xylopolyose dont dissous dans 5 ml de DMF anhydre. Cette solution est ensuite ajoutée très lentement au mélange précédent. Après 36h d'agitation à température ambiante, la solution est filtrée sur célite et le filtrat est évaporé sous pression réduite.

Le solide obtenu est enfin purifié par chromatographie sur colonne de silice (éluant : 20% MeOH / 80% DCM).

L'analyse par LC/MS montre que le mélange est constitué du composé **15** avec n = 0 à 4.

### Déprotection des fonctions de type acétal :

Sous atmosphère d'azote, 10 mg du composé **15** et 5 ml de méthanol anhydre sont introduits dans un ballon. Le mélange est refroidi à 0°C puis 100 µl d'une solution de méthylate de sodium à 1% (g/g) dans le méthanol est ajoutée goutte à goutte. Après quelques heures d'agitation de l'Amberlite IR120 est ajouté jusqu'à neutralisation du milieu. Après filtration de l'Amberlite, le filtrat est évaporé sous pression réduite pour donner le composé **16.**

### Exemple 12. Synthèse du substrat β-D-glucoside 5,6-benzo-7-hydroxycoumarin-4-méthanesulfonate de sodium (18)

### Greffage du sucre sur la coumarine :

Sous atmosphère d'azote et protection de la lumière, 80 mg de coumarine 4 dilués dans 6 ml de DMF anhydre et 5 équivalents de carbonate d'argent sont introduits dans un ballon. 5 équivalents d'acétobromo-α-D-glucose sont dissous dans 5 ml de DMF anhydre. Cette solution est ensuite ajoutée en 45 min au mélange précédent. Après 24h d'agitation à température ambiante, la solution est filtrée sur célite et le filtrat est évaporé sous pression réduite.

Le solide obtenu est purifié par chromatographie sur colonne de silice (éluant : 10% MeOH / 90% DCM). Le composé **17** est obtenu pur avec un rendement de 37%.
RMN ¹H (D₂O) δ (ppm) 8,04 (d, 1H) ; 7,50 (d, 1H) ; 7,25 (m, 2H) ; 6,56 (s, 1H) ; 6,31 (s, 1H) ; 5,30 (m, 2H) ; 5,08 (m, 1H) ; 4,96 (d, 1H) ; 4,56 (d, 1H) ; 4,26 (m, 1H) ; 4,21 (s, 2H) ; 4,03 (m, 1H) ; 2,10 (m, 12H).

### Déprotection des fonctions de type acétal :

Sous atmosphère d'azote, 60 mg du composé **17** et 15 ml de méthanol anhydre sont introduits dans un ballon. Le mélange est refroidi à 0°C puis 450 µl d'une solution de méthylate de sodium à 1% (g/g) dans le méthanol est ajoutée goutte à goutte. L'évolution de la réaction est suivie par LC/MS. Après 2h50 d'agitation, de l'Amberlite IR120 est ajouté jusqu'à neutralisation du milieu. Après filtration de l'Amberlite, le filtrat est évaporé sous pression réduite. Le résidu est repris dans de l'eau puis la solution est filtrée à 0,45 µm, le filtrat est de nouveau évaporé sous pression réduite.

Le résidu obtenu est purifié avec un appareil de purification *Biotage*-*IsoleraOne* (éluant : 5% acétonitrile dans l'eau pure, cartouche SNAP C18 30 g, débit d'élution : 10 ml/min).

Après évaporation des solvants, le produit **18** est obtenu pur avec un rendement de 33%.
RMN ¹H (D₂O) δ (ppm) 8,24 (d, 1H) ; 8,18 (d, 1H) ; 7,53 (m, 2H) ; 6,80 (s, 1H) ; 6,24 (s,1H) ; 5,25 (d, 1H) ; 4,49 (m,2H) ; 4,0-3,5 (m, 6H).

### Exemple 13. Synthèse du substrat β-D-xyloside 5,6-benzo-7-hydroxycoumarin-4-méthanesulfonate de sodium (20)

### Greffage du sucre sur la coumarine :

Sous atmosphère d'azote et protection de la lumière, 55 mg de coumarine **4** dilués dans 4 ml de DMF anhydre et 5 équivalents de carbonate d'argent (0,3 g) sont introduits dans un ballon. 5 équivalents d'acétobromo-α-D-xylose (obtenu comme décrit dans l'exemple 9) sont dissous dans 4 ml de DMF anhydre. Cette solution est ensuite ajoutée lentement au mélange précédent. Après 20h d'agitation à température ambiante, la solution est filtrée sur célite et le filtrat est évaporé sous pression réduite.

Le solide obtenu est purifié par chromatographie sur colonne de silice (éluant : 10% MeOH / 90% DCM). Le composé **19** est obtenu pur avec un rendement de 42%.
RMN ¹H (MeOD/D₂O) δ (ppm) 8,57 (d, 1H) ; 8,00 (d, 1H) ; 7,68 (t, 1H) ; 7,55 (t, 1H) ; 6,89 (s, 1H) ; 6,52 (s, 1H) ; 5,53 (m, 1H) ; 5,31 (m, 2H) ; 5,08 (m, 1H) ; 4,61 (m, 2H) ; 4,23 (m, 1H) ; 3,78 (m, 1H) ; 2,17 (m, 9H).

### Déprotection des fonctions de type acétal :

Sous atmosphère d'azote, 40 mg du composé **19** et 11 ml de méthanol anhydre sont introduits dans un ballon. Le mélange est refroidi à 0°C puis 500 µl d'une solution de méthylate de sodium à 1% (g/g) dans le méthanol est ajoutée goutte à goutte. Après 2h15 de réaction, 400 µl de la solution de méthylate de sodium sont de nouveau ajoutés. Après 3h30 d'agitation, de l'Amberlite IR120 est ajouté jusqu'à neutralisation du milieu. Après filtration de l'Amberlite, le filtrat est évaporé sous pression réduite.

Le résidu obtenu est purifié par chromatographie sur colonne de silice inverse pré-packée (éluant : 10% acétonitrile dans l'eau pure, cartouche *Puriflash Interchrom* C18) pour donner le composé 20 avec un rendement de 35%.
RMN ¹H (D₂O) δ (ppm) 8,09 (d, 1H) ; 7,99 (d, 1H) ; 7,46 (m, 1H) ; 7,38 (m, 1H) ; 6,51 (s, 1H) ; 6,16 (s, 1H) ; 4,98 (m, 1H) ; 4,37 (m, 2H) ; 4,01 (m, 1H) ; 3,8-3,4 (m, 4H).

### Exemple 14. Synthèse du substrat β-D-glucoside 7-hydroxy-8-méthylcoumarin-4-méthanesulfonate de sodium (22)

### Greffage du sucre sur la coumarine :

Sous atmosphère d'azote et protection de la lumière, 80 mg de coumarine 6 dilués dans 7 ml de DMF anhydre et 5 équivalents de carbonate d'argent (377 mg) sont introduits dans un ballon. 5 équivalents (563 mg) d'acétobromo-α-D-glucose sont dissous dans 7 ml de DMF anhydre. Cette solution est ensuite ajoutée lentement au mélange précédent. Après 20h d'agitation à température ambiante, la solution est filtrée sur célite et le filtrat est évaporé sous pression réduite.

Le solide obtenu est purifié une première fois par chromatographie sur colonne de silice (éluant : 10% MeOH / 90% DCM) puis par chromatographie sur colonne de silice inverse pré-packée (éluant : gradient de 0% à 50% d'acétonitrile dans l'eau pure, cartouche *Puriflash Interchrom* C18). Le composé **21** est ainsi obtenu pur avec un rendement de 17%.
RMN ¹H (MeOD) δ (ppm) 7,85 (d, 1H) ; 7,67 (d, 1H) ; 6,47 (s, 1H) ; 5,47 (m, 2H) ; 5,30 (t, 1H) ; 5,16 (t, 1H) ; 4,4-4,1 (m, 5R) ; 2,25 (s, 3H) ; 2,08 (m, 12H).

### Déprotection des fonctions de type acétal :

Sous atmosphère d'azote, 18 mg du composé **21** et 5 ml de méthanol anhydre sont introduits dans un ballon. Le mélange est refroidi à 0°C puis 100 µl d'une solution de méthylate de sodium à 1% (g/g) dans le méthanol est ajoutée goutte à goutte. L'évolution de la réaction est suivie par CCM. Apres 1h30 d'agitation, de l'Amberlite IR120 est ajouté jusqu'à neutralisation du milieu. Après filtration de l'Amberlite, le filtrat est évaporé sous pression réduite.

Le composé **22** est obtenu avec un rendement de 34%.
RMN ¹H (D₂O) δ (ppm) 7,53 (d, 1H) ; 7,06 (d, 1H) ; 6,26 (s, 1H) ; 5,15 (d, 1H) ; 4,24 (m, 2H) ; 3,90 (m, 1H) ; 3,73 (m, 1H) ; 3,6-3,4 (m, 4H) ; 2,13 (s, 3H).

### Exemple 15. Synthèse du substrat β-D-galactoside 7-hydroxy-8-méthylcoumarin-4-méthanesulfonate de sodium (24)

### Greffage du sucre sur la coumarine :

Sous atmosphère d'azote et protection de la lumière, 80 mg de coumarine **6** dilués dans 5 ml de DMF anhydre et 5 équivalents de carbonate d'argent (377 mg) sont introduits dans un ballon. 5 équivalents (563 mg) d'acétobromo-α-D-galactose sont dissous dans 5 ml de DMF anhydre. Cette solution est ensuite ajoutée lentement au mélange précédent. L'évolution de la réaction est suivie par CCM. Après 40h d'agitation à température ambiante, la solution est filtrée sur célite et le filtrat est évaporé sous pression réduite.

Le solide obtenu est purifié une première fois par chromatographie sur colonne de silice inverse pré-packée (éluant : gradient de 0% à 50% d'acétonitrile dans l'eau pure, cartouche *Puriflash Interchrom* C18) puis par chromatographie sur colonne de silice (éluant : 10% MeOH / 90% DCM). Le composé **23** est ainsi obtenu pur avec un rendement de 42%.
RMN ¹H (MeOD) δ (ppm) 7,81 (d, 1H) ; 7,65 (d, 1H) ; 6,45 (s, 1H) ; 5,49 (m, 3H) ; 5,37 (m, 1H) ; 4,37 (m, 3H) ; 4,20 (m, 2H) ; 2,23 (s, 3H) ; 2,19 (s, 3H) ; 2,12 (s, 3H) ; 2,08 (s, 3H) ; 2,03 (s, 3H). RMN ¹³C (MeOD) δ (ppm) 170,80 ; 170,65 ; 170,08 ; 169,97 ; 161 ,57 ; 157,33 ; 152,52 ; 148,64 ; 124,58 ; 114,82 ; 114,41 ; 114,15 ; 110,77 ; 98,48 ; 70,93 ; 70,67 ; 68,60 ; 67,39 ; 61,29 ; 52,61 ; 19,41 ; 19,37 ; 19,19 ; 19,18 ; 7,03.

### Déprotection des fonctions de type acétal :

Sous atmosphère d'azote, 45 mg du composé **23** et 20 ml de méthanol anhydre sont introduits dans un ballon. Le mélange est refroidi à 0°C puis 300 µl d'une solution de méthylate de sodium à 1% (g/g) dans le méthanol est ajoutée goutte à goutte. L'évolution de la réaction est suivie par CCM. Apres 1h30 d'agitation, la réaction n'est pas totale, 300 µl de la solution de méthylate sont de nouveau ajoutés. Après 3h de réaction, de l'Amberlite IR120 est ajouté jusqu'à neutralisation du milieu. Après filtration de l'Amberlite, le filtrat est évaporé sous pression réduite.

Le composé **24** est obtenu avec un rendement de 36%.
RMN ¹H (D₂O) δ (ppm) 7,51 (d, 1H) ; 7,07 (d, 1H) ; 6,24 (s, 1H) ; 5,09 (d, 1H) ; 4,0-3,6 (m, 8H) ; 2,13 (s, 3H).

Les substrats ainsi obtenus trouvent notamment leur application pour la détection d'activités glycosidases (EC3.2.1) sur des extraits enzymatiques purifiés ou non ou sur des microorganismes ou sur des cellules.

Il sera par exemple possible d'effectuer le dosage fluorescent d'activités xylanase et/ou cellulase et/ou cellobiase et/ou glucosidase et/ou xylosidase et/ou galactosidase d'extraits enzymatiques, ou bien d'effectuer le criblage par fluorescence selon leurs activités glycosidases (EC3.2.1) d'enzymes ou de microorganismes ou des cellules.

De façon plus particulière, les nouveaux substrats selon l'invention permettront la détection d'activités glycosidases (EC3.2.1) sur des extraits enzymatiques purifiés ou non ou sur des microorganismes ou sur des cellules compartimentés dans des gouttelettes aqueuses en suspension dans une phase huile (émulsion) produites par agitation mécanique. Il sera ainsi possible de réaliser le criblage par FACS (*Fluorescence Activated Cell Sorting*) selon leurs activités xylanase et/ou cellulase et/ou cellobiase et/ou glucosidase et/ou xylosidase et/ou galactosidase d'enzymes ou de microorganismes ou de cellules compartimentés dans des gouttelettes aqueuses en suspension dans une phase huile.

De façon encore plus particulière, les substrats selon l'invention permettront la détection d'activités glycosidases (EC3.2.1) sur des extraits enzymatiques purifiés ou non ou sur des microorganismes ou sur des cellules compartimentés dans des gouttelettes aqueuses en suspension dans une phase huile (émulsion) produites par un dispositif microfluidique. Dans cette hypothèse, il sera possible de réaliser par exemple le criblage selon leurs activités xylanase et/ou cellulase et/ou cellobiase et/ou glucosidase et/ou xylosidase et/ou galactosidase d'enzymes ou de microorganismes ou de cellules compartimentés dans des gouttelettes aqueuses en suspension dans une phase huile créée par un dispositif microfluidique.

Différents exemples de détections d'activités enzymatiques à l'aide des substrats fluorogéniques décrits vont maintenant être donnés, à titre indicatif et nullement limitatif.

### Exemple 16. Détection d'activités enzymatiques sur extraits enzymatiques solubles avec les substrats 10, 12, 14 et 18

Les cinétiques enzymatiques sont réalisées en microplaque sur des extraits enzymatiques solubles (A, B, C et D) riches en activités de type cellulase et xylanase. Ces extraits sont dilués 10 fois (substrat **18,** Figure 1) ou 100 fois (substrat **10, 12** et **14,** Figure 2) dans du tampon phosphate 50 mM, NaCl 150 mM, pH = 7,5. Un volume de 20 µL, d'extrait enzymatique dilué est ajouté à 20 µL de substrat fluorogénique (**10, 12, 14** ou **18**) à 0,25 mM dans du tampon phosphate 50 mM, NaCl 150 mM, pH = 7,5. Les cinétiques sont réalisées à 30°C. L'hydrolyse enzymatique des différents substrats fluorogéniques est suivie pendant 120 minutes par mesure de la fluorescence avec des longueurs d'excitation et d'émission de : 339 nm et 452 nm pour les substrats **10, 12** et **14 ;** 375 nm et 510 nm pour le substrat **18.**

Les résultats obtenus (Figures 1 et 2) montrent que les substrats fluorogéniques testés permettent de détecter des activités enzymatiques de type β-glucosidase (substrat **10** et **18**), β-xylosidase (substrat **12**) et xylanase (substrat **14**) dans les extraits enzymatiques utilisés.

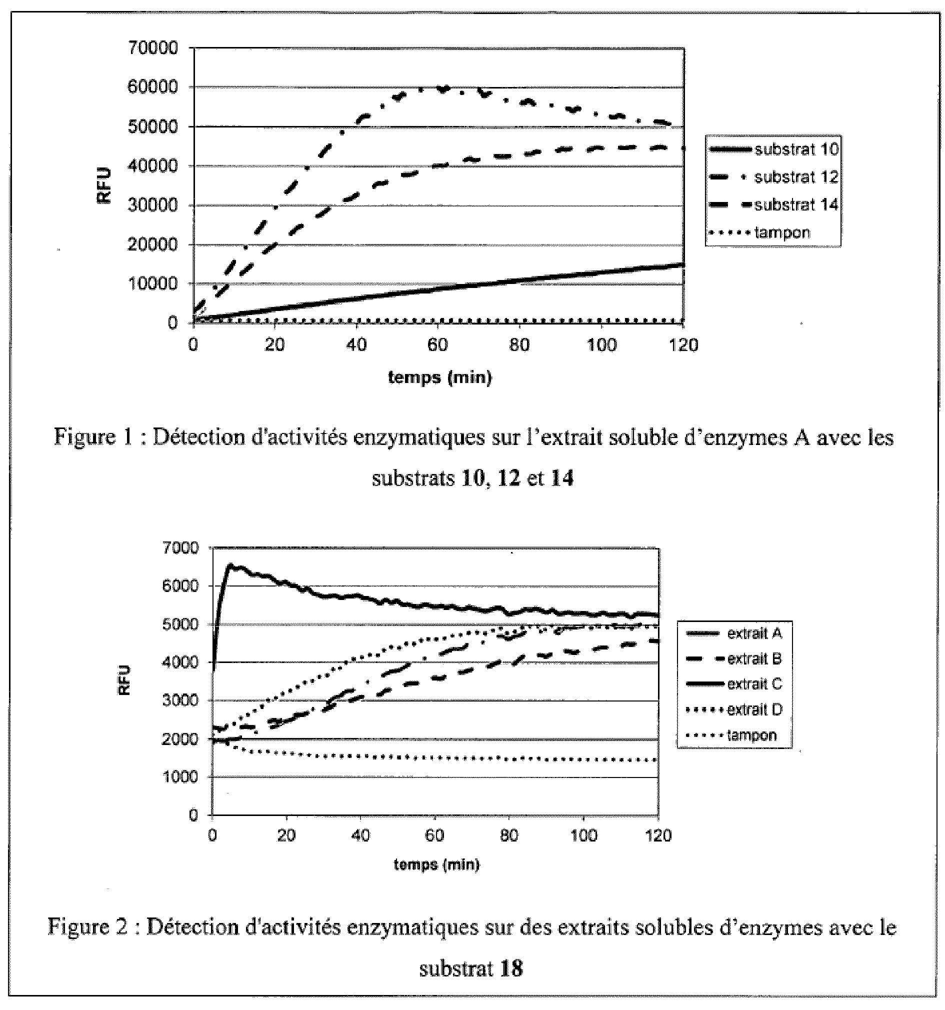

### Exemple 17. Détection d'activités enzymatiques sur microorganismes avec les substrats fluorogéniques 8 et 10

Différentes souches témoins sont mises en culture en milieu LB à 30°C sous agitation (240 rpm) pendant 18h. Pour chaque souche, la culture est centrifugée (2 500 g, 5 min) et le culot de cellules est lavé deux fois dans 5 ml de milieu LB (centrifugation à 2 500 g, 5 min). Ce culot est utilisé pour inoculer à DO = 0,005 une culture en milieu inducteur (milieu Dubos à 2,5 g/L de carboxymethyl-cellulose) contenant le substrat fluorogénique (**8** ou **10**) à 0,25 mM. La culture est incubée à 30°C sans agitation pendant 24h. Des échantillons de surnageant de culture sont prélevés à différents temps d'incubation pour pouvoir suivre l'apparition des activités cellulase ou β-glycosidase dans le milieu de culture. L'apparition de ces activités est suivie par mesure de la fluorescence sur les surnageants de culture en microplaques avec des longueurs d'onde d'excitation et d'émission de 388 nm et 455 nm.

Les résultats obtenus (Figures 3 et 4) montrent que les substrats fluorogéniques testés permettent de détecter des activités enzymatiques de type β-glycosidase (substrat **10**) et cellulase (substrat **8**) sur des cultures de microorganismes.

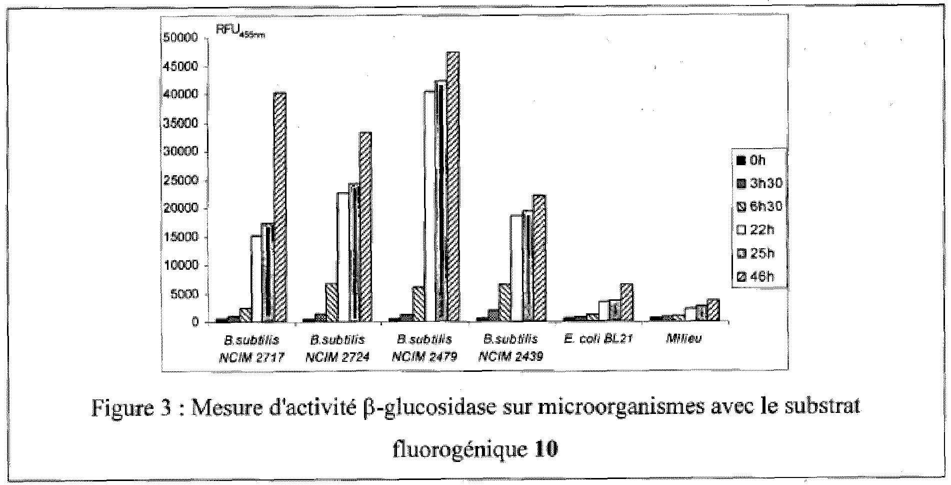

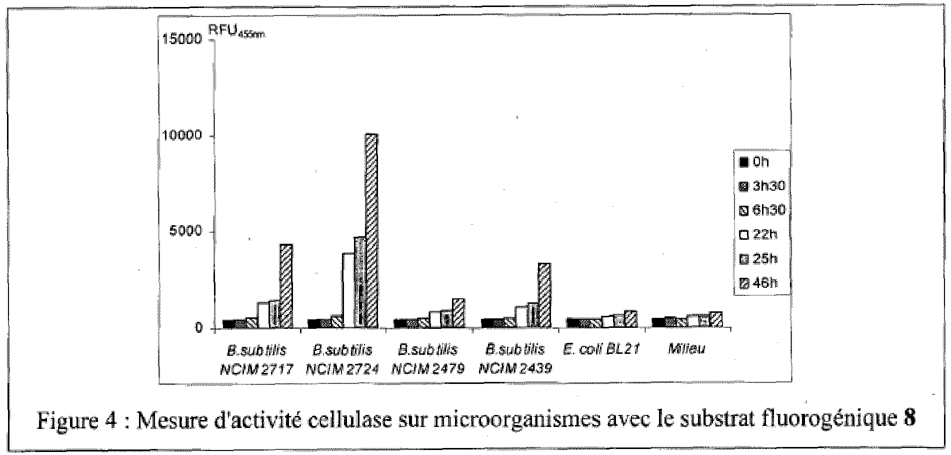

### Exemple 18. Détection d'activité cellulase sur microorganismes en émulsion avec le substrat 8

Deux souches témoins sont utilisées : l'une présentant une activité cellulase (*Bacillus subtilis NCIM* 2724) l'autre n'en présentant aucune (*Escherichia coli BL21*). Les deux souches sont mises en culture en milieu LB à 30°C sous agitation (240 rpm) pendant 18h. Pour chaque souche, la culture est centrifugée (2 500 g, 5 min) et le culot de cellules est lavé deux fois dans 5 ml de milieu LB (centrifugation à 2 500 g, 5 min). Ce culot est utilisé pour inoculer à DO = 0,005 une culture en milieu inducteur (milieu Dubos à 2,5 g/L de carboxymethyl-cellulose) contenant le substrat fluorogénique **8** et 2,5 µM (*B*. *subtilis NCIM2724*) ou 10 µM (*E*.*coli BL21*) de sulforhodamine. Ces deux suspensions de cellules sont utilisées avec de l'huile perfluorée pour produire une émulsion composée de deux populations de gouttelettes contenant chacune l'une des suspensions décrites. Les fluorescences bleue et rouge des gouttelettes sont mesurées individuellement, sur un échantillon de l'émulsion, lors de la production (t = Oh) et de la réinjection après avoir incubé l'émulsion 24h à 30°C.

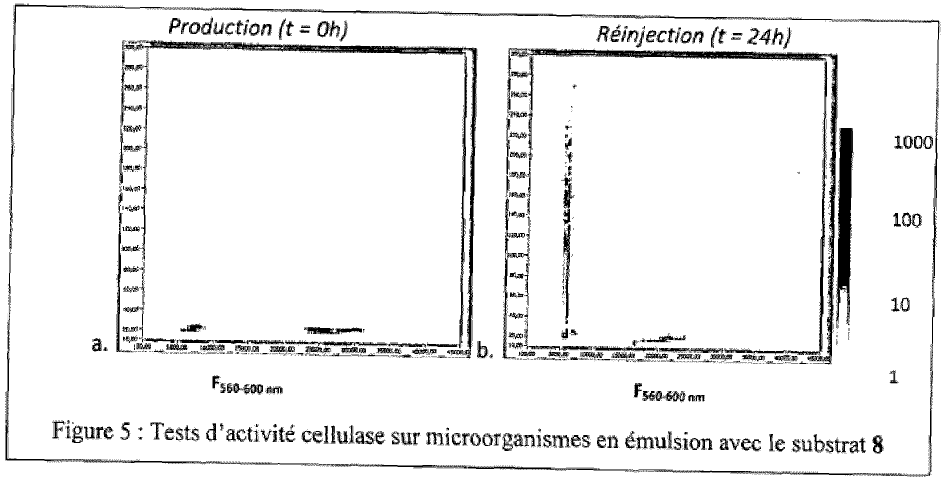

Les graphiques a et b, présentés Figure 5, sont les histogrammes à deux dimensions des intensités de fluorescence rouge (en abscisse, *RFU*) et bleue (en ordonnée, *RFU*) mesurées sur l'émulsion lors de sa production (a) et de sa réinjection après incubation pendant 24 h à 30°C (b). La densité de population est représentée par un code couleur variant du rose (1 gouttelette) au rouge (>1000 gouttelettes) selon une échelle logarithmique. Deux populations de gouttelettes sont observables : l'une à faible fluorescence rouge contenant *Bacillus subtilis NCIM 2724* (souche active) et 2,5 µM de sulforhodamine, l'autre à fluorescence rouge intense contenant *Escherichia coli BL21* (souche inactive) et 10 µM de sulforhodamine). L'analyse statistique de ces histogrammes est présentée dans le Tableau 1.

**Tableau 1 : Analyse statistique des populations de gouttelettes lors du test d'activité cellulase sur microorganismes en émulsion avec le substrat 8**

| | [sulforhodamine] | 2,5 µM | 10 µM |
|---|---|---|---|
| | **Identité des souches** | ***B.subtilis NCIM 2724*** | ***E***.***coli BL21*** |
| Production (t = 0h) | Nombre de gouttes analysées | 16083 | |
| | Fluorescence rouge moyenne (RFU) | 6 434 ±387 | 25 775 ±825 |
| | Fluorescence bleue moyenne (RFU) | 18,78 ±,19 | |
| | **Seuil de détection des goutelettes positives (RFU)** | **37,56** | |
| Réinjection (t = 24h) | Nombre de gouttes analysées | 15 869 | |
| | **% de gouttelettes positives** | **27**,**61**% | **0,26%** |

Les gouttelettes contenant la souche active (*Bacillus subtilis NCIM 2724*) voient leur fluorescence bleue significativement augmentée après 24h d'incubation (Figure 5). À l'inverse les gouttelettes contenant la souche inactive (*Escherichia coli BL21*) ne présentent aucune augmentation de leur fluorescence bleue. Pour la souche active (*Bacillus subtilis NCIM 2724*), 27% des gouttelettes sont identifiées comme positive, contre 0,29% pour la souche inactive (*Escherichia coli BL21*). En conclusion, le substrat **8** permet de détecter une activité de type cellulase sur des microorganismes au sein d'une émulsion générée par un système microfluidique.

## Revendications

1. Substrat fluorogénique ayant pour formule générale (II) 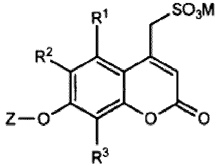 dans laquelle :
- R¹ représente H, ou OH, ou un radical alkyle de C₁ à C₆ substitué ou non, linéaire ou ramifié, ou -COR⁴, ou -COOR⁴, ou -CONHR⁴
- R² représente H, ou un halogène, notamment le fluor, ou un radical alkyle de C₁ à C₆ substitué ou non, linéaire ou ramifié, ou -COR⁴, ou -COOR⁴, ou -CONHR⁴
- R¹ et R² pouvant former ensemble un cycle, tel qu'un aryle ou un furane, substitué ou non
- R³ représente H, ou un halogène, notamment le fluor, ou un radical alkyle de C₁ à C₆ substitué ou non, linéaire ou ramifié, ou -COR⁴, ou -COOR⁴, ou -CONHR⁴
- avec R⁴ étant H, ou un radical alkyle de C₁ à C₆ substitué ou non, linéaire ou ramifié ou un aryle, substitué ou non
- M représente Na ou K
- Z représente un sucre choisi parmi les sucres suivants : cellobiose, xylobiose, maltose, saccharose, glucose, xylose, galactose, arabinose, xylane, glucane, xylotriose, maltotriose, cellotriose, xylotétraose ou un mélange de certains d'entre eux
- et dans laquelle Z ne pouvant être le galactose lorsque R¹ est H, R² et R³ sont F et M est Na.

2. Substrat selon la revendication 1 **caractérisé en ce qu'**il fait partie du groupe suivant :
β-D-cellobioside 6,8-difluoro-7-hydroxycoumarin-4-méthanesulfonate de sodium, β-D-glucoside 6,8-difluoro-7-hydroxycoumarin-4-méthanesulfonate de sodium, β-D-xyloside 6,8-difluoro-7-hydroxycoumarin-4-méthanesulfonate de sodium, β-D-xylobioside 6,8-difluoro-7-hydroxycoumarin-4-méthanesulfonate de sodium, β-D-xylopolyoside 6,8-difluoro-7-hydroxycoumarin-4-méthanesulfonate de sodium, β-D-glucoside 5,6-benzo-7-hydroxycoumarin-4-méthanesulfonate de sodium, β-D-xyloside 5,6-benzo-7-hydroxycoumarin-4-méthanesulfonate de sodium, β-D-glucoside 7-hydroxy-8-méthylcoumarin-4-méthanesulfonate de sodium, β-D-galactoside 7-hydroxy-8-méthylcoumarin-4-méthanesulfonate de sodium.

3. Procédé d'obtention d'un substrat selon l'une des revendications 1 ou 2 **caractérisé en ce que** le greffage du sucre sur la coumarine est réalisé dans du DMF.

4. Procédé selon la revendication 3 **caractérisé en ce que** ledit greffage est suivi d'une déprotection des fonctions de type acétal.

5. Procédé selon l'une des revendications 3 ou 4 **caractérisé en ce que** ledit sucre est bromé avant d'être greffé sur ladite coumarine.

6. Procédé selon l'une quelconque des revendications 3 à 5 **caractérisé en ce que** la purification du substrat est réalisée par chromatographie d'absorption sur colonne de silice en phase inverse.

7. Procédé selon l'une quelconque des revendications 3 à 6, **caractérisé en ce que** le substrat est obtenu par greffage d'un sucre sur une coumarine sulfonatée comprenant la réaction du dérivé acétobromo dudit sucre avec la coumarine sulfonatée dans le DMF, puis la déprotection des fonctions du type acétal.

8. Détection d'activités glycosidases (EC3.2.1) sur des extraits enzymatiques purifiés ou non ou sur des microorganismes ou sur des cellules **caractérisée en ce que** ladite détection se fait grâce à l'un des substrats fluorogéniques selon l'une des revendications 1 ou 2.

9. Détections d'activités glycosidases (EC3.2.1) sur des extraits enzymatiques purifiés ou non ou sur des microorganismes ou sur des cellules selon la revendication 8 **caractérisée en ce que** lesdits extraits ou microorganismes ou cellules sont compartimentés dans des gouttelettes aqueuses en suspension dans une phase huile.

10. Détection d'activités glycosidases (EC3.2.1) sur des extraits enzymatiques purifiés ou non ou sur des microorganismes ou sur des cellules selon la revendication 9 **caractérisée en ce que** lesdites gouttelettes aqueuses sont produites par un dispositif microfluidique.

## Patentansprüche

1. Fluorogenes Substrat mit der allgemeinen Formel (II) 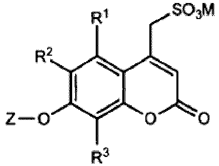 wobei:
- R¹, H oder OH oder ein C₁-C₆-Alkylradikal, substituiert oder nicht, linear oder verzweigt, oder -COR⁴ oder -COOR⁴ oder -CONHR⁴ darstellt,
- R² H oder ein Halogen, insbesondere Fluor, oder ein C₁-C₆-Alkylradikal, substituiert oder nicht, linear oder verzweigt, oder -COR⁴ oder -COOR⁴ oder -CONHR⁴ darstellt,
- R¹ und R² gemeinsam einen Ring wie ein Aryl oder ein Furan, substituiert oder nicht, bilden können,
- R³ H oder ein Halogen, insbesondere Fluor, oder ein C₁-C₆-Alkylradikal, substituiert oder nicht, linear oder verzweigt, oder -COR⁴ oder -COOR⁴ oder -CONHR⁴ darstellt,
- mit R⁴ als H oder ein C₁-C₆-Alkylradikal, substituiert oder nicht, linear oder verzweigt, oder ein Aryl, substituiert oder nicht,
- M Na oder K darstellt,
- Z einen Zucker darstellt, ausgewählt aus den folgenden Zuckern: Cellobiose, Xylobiose, Maltose, Saccharose, Glucose, Xylose, Galactose, Arabinose, Xylan, Glucan, Xylotriose, Maltotriose, Cellotriose, Xylotetraose oder einem Gemisch einiger von ihnen,
- und wobei Z keine Galactose sein kann, wenn R¹ H ist, R² und R³ F sind und M Na ist.

2. Substrat nach Anspruch 1, **dadurch gekennzeichnet, dass** es zu der folgenden Gruppe gehört:
Natrium-β-D-cellobiosid 6,8-difluoro-7-hydroxycoumarin-4-methansulfonat, Natrium-β-D-glucosid 6,8-difluoro-7-hydroxycoumarin-4-methansulfonat, Natrium-β-D-xylosid 6,8-difluoro-7-hydroxycoumarin-4-methansulfonat, Natrium-β-D-xylobiosid 6,8-difluoro-7-hydroxycoumarin-4-methansulfonat, Natrium-β-D-xylopolyosid 6,8-difluoro-7-hydroxycoumarin-4-methansulfonat, Natrium-β-D-glucosid 5,6-benzo-7-hydroxycoumarin-4-methansulfonat, Natrium-β-D-xylosid 5,6-benzo-7-hydroxycoumarin-4-methansulfonat, Natrium-β-D-glucosid 7-hydroxy-8-methylcoumarin-4-methansulfonat, Natrium-β-D-galactosid 7-hydroxy-8-methylcoumarin-4-methansulfonat.

3. Verfahren zur Herstellung eines Substrats nach einem der Ansprüche 1 oder 2, **dadurch gekennzeichnet, dass** das Pfropfen des Zuckers auf das Coumarin in DMF durchgeführt wird.

4. Verfahren nach Anspruch 3, **dadurch gekennzeichnet, dass** dem Pfropfen eine Entschützung der Funktionen vom Typ Acetal folgt.

5. Verfahren nach einem der Ansprüche 3 oder 4, **dadurch gekennzeichnet, dass** der Zucker bromiert ist, bevor er auf das Coumarin gepfropft wird.

6. Verfahren nach einem der Ansprüche 3 bis 5, **dadurch gekennzeichnet, dass** die Reinigung des Substrats durch Absorptionschromatographie auf Siliciumdioxidsäule in Umkehrphase durchgeführt wird.

7. Verfahren nach einem der Ansprüche 3 bis 6, **dadurch gekennzeichnet, dass** das Substrat durch Pfropfen eines Zuckers auf ein sulfoniertes Coumarin hergestellt wird, umfassend die Reaktion des Acetobromderivats des Zuckers mit dem sulfonierten Coumarin in DMF, gefolgt von der Entschützung der Funktionen vom Typ Acetal.

8. Detektion von Glycosidase-Aktivitäten (EC3.2.1) auf enzymatischen Extrakten, gereinigt oder nicht, oder auf Mikroorganismen oder auf Zellen, **dadurch gekennzeichnet, dass** die Detektion dank eines der fluorogenen Substrate nach einem der Ansprüche 1 oder 2 erfolgt.

9. Detektion von Glycosidase-Aktivitäten (EC3.2.1) auf enzymatischen Extrakten, gereinigt oder nicht, oder auf Mikroorganismen oder auf Zellen nach Anspruch 8, **dadurch gekennzeichnet, dass** die Extrakte oder Mikroorganismen oder Zellen in wässrigen Tröpfchen in Suspension in einer Ölphase kompartimentiert sind.

10. Detektion von Glycosidase-Aktivitäten (EC3.2.1) auf enzymatischen Extrakten, gereinigt oder nicht, oder auf Mikroorganismen oder auf Zellen nach Anspruch 9, **dadurch gekennzeichnet, dass** die wässrigen Tröpfen von einer Mikrofluidvorrichtung produziert sind.

## Claims

1. A fluorogenic substrate having the general formula (II) 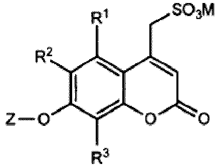 wherein:
- R¹ represents H, or OH, or a linear or branched C₁-C₆ alkyl radical, either substituted or not, or -COR⁴, or -COOR⁴, or -CONHR⁴,
- R² represents H, or a halogen, notably fluorine, or a linear or branched C₁-C₆ alkyl radical, either substituted or not, or -COR⁴, or -COOR⁴, or -CONHR⁴,
- R¹ and R² may form together a ring, such as an aryl or a furane, either substituted or not,
- R³ represents H, or a halogen, notably fluorine, or a linear or branched C₁-C₆ alkyl radical, either substituted or not or -COR⁴, or -COOR⁴, or -CONHR⁴,
- with R⁴ being H, or a linear or branched C₁-C₆ alkyl radical either substituted or not or an aryl either substituted or not,
- M represents Na or K,
- Z represents a sugar selected from the following sugars: cellobiose, xylobiose, maltose, saccharose, glucose, xylose, galactose, arabinose, xylan, glucan, xylotriose, maltotriose, cellotriose, xylotetraose or a mixture of some of them,
- and wherein Z cannot be galactose when R¹ is H, R² and R³ are F and M is Na.

2. The substrate according to claim 1 **characterized in that** it belongs to the following group:
sodium β-D-cellobioside 6,8-difluoro-7-hydroxycoumarin-4-methanesulfonate, sodium β-D-glucoside 6,8-difluoro-7-hydroxycoumarin-4-methanesulfonate, sodium β-D-xyloside 6,8-difluoro-7-hydroxycoumarin-4-methanesulfonate, sodium β-D-xylobioside 6,8-difluoro-7-hydroxycoumarin-4-methanesulfonate, sodium β-D-xylopolyoside 6,8-difluoro-7-hydroxycoumarin-4-methanesulfonate, sodium β-D-glucoside 5,6-benzo-7-hydroxycoumarin-4-methanesulfonate, sodium β-D-xyloside 5,6-benzo-7-hydroxycoumarin-4-methanesulfonate, sodium β-D-glucoside 7-hydroxy-8-methylcoumarin-4-methanesulfonate, sodium β-D-galactoside 7-hydroxy-8-methylcoumarin-4-methanesulfonate.

3. A method for obtaining a substrate according to one of claims 1 or 2, **characterized in that** the grafting of the sugar on the coumarin is achieved in DMF.

4. The method according to claim 3 **characterized in that** said grafting is followed by deprotection of the functions of the acetal type.

5. The method according to one of claims 3 or 4, **characterized in that** said sugar is brominated before being grafted on said coumarin.

6. The method according to any of claims 3 to 5, **characterized in that** the purification of the substrate is achieved by reverse phase absorption chromatography on a silica column.

7. The method according to any of claims 3 to 6, **characterized in that** the substrate is obtained by grafting a sugar onto a sulfonated coumarin comprising reacting the acetobromo derivative of said sugar with the sulfonated coumarin in the DMF, then deprotection of the functions of the acetal type.

8. A detection of glycosidase activities (EC3.2.1) on enzymatic extracts either purified or not or on microorganism or on cells, **characterized in that** said detection is accomplished by means of one of the fluorogenic substrates according to one of claims 1 or 2.

9. The detection of glycosidase activities (EC3.2.1) on enzymatic extracts either purified or not or on microorganisms or on cells according to claim 8, **characterized in that** said extracts or microorganisms or cells are compartmented in aqueous droplets in suspension in an oil phase.

10. The detection of glycosidase activities (EC3.2.1) on enzymatic extracts either purified or not or on microorganisms or on cells according to claim 9, **characterized in that** said aqueous droplets are produced by a micro fluidic device.
